# EUROPEAN PATENT APPLICATION

(11) **EP 1 972 264 A1**
(43) Date of publication of application: **24.09.2008**
(21) Application number: 08250452.3
(22) Date of filing: 07.02.2008
(51) Int. Cl.: A61B 1/313, A61B 19/00, F16M 11/14, F16C 11/10

(54) **Endoscopic instrument holder**

(30) Priority: 07.02.2007 US 888637 P
(71) Applicant: Codman & Shurtleff, Inc., Raynham, Massachusetts 02767-0350 (US)
(72) Inventor: Agbodoe, Victor, Stoughton, MA 02072 (US); Storz, Olaf, 78532 Tuttlingen (DE); Richardson, Gary, Brockton, MA 02302 (US)
(74) Representative: Tunstall, Christopher Stephen

(57) **Abstract**

An endoscopic instrument holder includes an elongated bar having a proximal end and a distal end. A pivoting lever is disposed at the proximal end. A grasping mechanism and a handle are disposed at the distal end. A selectively flexible wire and ball joint assembly is disposed between the proximal end and the distal end. The lever is moveable to selectively place the bar in one of a fixed position and an adjustable position. In the adjustable position the bar is free to move into a position determined by a user. In the fixed position the bar is rigid. The grasping mechanism has a fixed clamp portion and a moveable clamp portion. A handle is disposed at the distal end of the bar. Actuation of the handle causes movement of the moveable clamp portion with respect to the fixed clamp portion.

## Description

### FIELD OF THE INVENTION

The present invention relates to methods and devices for holding an endoscopic instrument.

### BACKGROUND OF THE INVENTION

During surgery, retractors, such as the those found in the BOOKWALTERT^{™} retractor kit, which is commercially available from Codman & Shurtleff, Inc. of Raynham, MA, are often used to assist the surgeon and other operating room personnel to provide exposure to the surgical site for a broad range of surgical procedures. In surgical operations of the chest or abdomen for example, it is often necessary to use a retraction apparatus to retain tissue away from the operative site. Typically, the retraction apparatus includes a housing member configured to lock onto a circumferential ring located above the operative site. To maintain the ring in a fixed position, the ring can be connected to a support post adjacent to the site. An extension arm may be attached to the support post for supporting the circumferential ring. Within the housing member a retraction blade can usually be found for grabbing the tissue around the surgical incision. The housing member can also include a ratcheting mechanism and/or a tilting mechanism to draw the retraction blade away from the incision, thereby effecting the pulling away and/or lifting of the tissue around the incision to expose the desired surgical area. Examples of such retractor systems are disclosed in U.S. Pat. Nos. 4,254,763, 4,424,724 and 5,375,481, the disclosures of which are hereby incorporated by reference. During open surgical operations, such as, for example, open bariatric, ALIF procedures, hepatic resections, transplant procedures, abdominal aortic aneurysms, hernia repair, appendectomy and others, many different instruments are used, such as, for example, a retractor blade with ring attachment systems are used. Laporoscopic instruments, fiber optic cables and endoscopic instruments are used for small, or minimally invasive operations, hereinafter referred to as endoscopic instruments. Typically, however, these endoscopic instruments are held by hand by one of the physician's assistants. Human fatigue factor often results in unintended movement or drift of the endoscopic instrument.

Thus, despite these retraction systems, there continues to be a need for a retraction system that can securely hold an endoscopic instrument and permit the quick positioning of that instrument with respect to the surgical area. It would therefore be advantageous to have an endoscopic instrument holder that provides the surgeon with even greater control over the placement of the endoscopic instrument with respect to the exposure of the surgical area.

### SUMMARY OF THE INVENTION

### BRIEF DESCRIPTION OF THE DRAWINGS

The invention will be more fully understood from the following detailed description taken in conjunction with the accompanying drawings, in which:

FIG. 1 is a plan view of a holder for endoscopic instruments;

FIG. 2 is a partial view of the lever in the open position;

FIG. 3 is a partial view of the lever in the closed position;

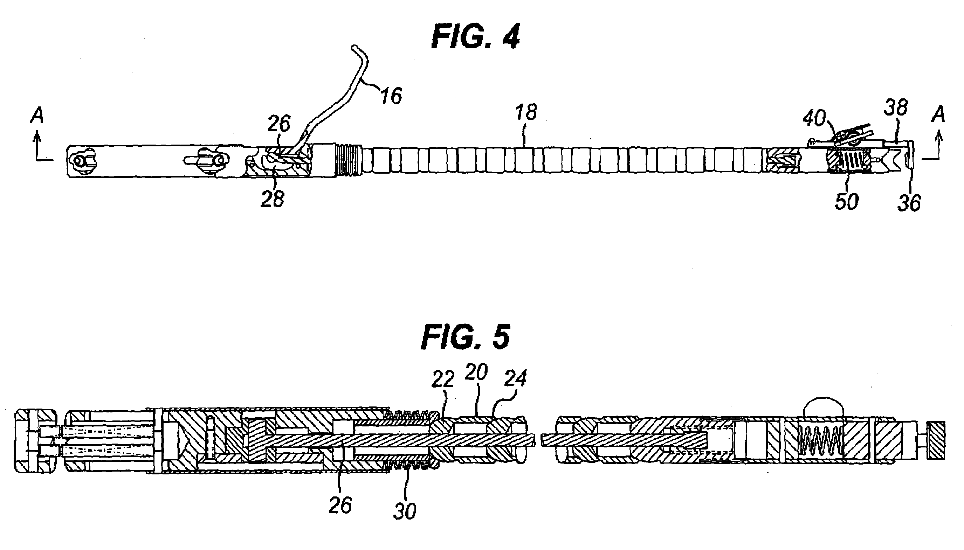

FIG. 4 is a plan view similar to Fig. 1, but with parts broken away;

FIG. 5 is a cross-sectional view taken along lines "A"-"A" of Fig. 4 and looking in the direction of the arrows;

FIG. 6 is a partial sectional view taken along lines "B"-"B" of Fig. 7 and looking in the direction of the arrows; and

FIG. 7 is an enlarged partial view of the quick release mechanism.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

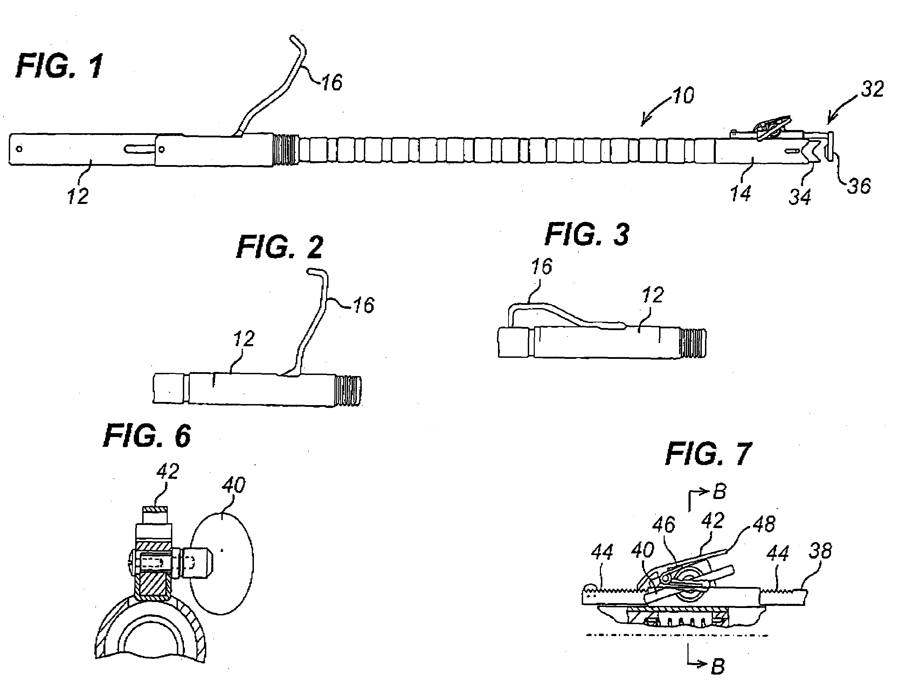

The present invention provides methods and devices for holding an endoscopic instrument. An endoscopic instrument holder 10 is illustrated in Fig. 1. Holder 10 has a proximal end 12 and a distal end 14. A pivoting lever 16 is disposed at proximal end 12 and moves between an open position as shown in Figs. 1 and 2 and a closed position shown in Fig. 3. Intermediate of proximal end 12 and distal end 14 is a selectively flexible steel cable wire and ball joints 18. Steel cable wire and ball joints 18 are comprised of a plurality of interlocking ball joint elements 20, each of which is ball-shaped at one end 22 and has a ball-receiving portion 24 at the other end. With lever 16 in the open position, a cable 26 provides sufficient slack to permit snake 18 to adjust into any position desired by the user. Once in the desired position, the user can move lever 16 to the closed position, as shown in Fig. 3, which causes cable 26 to tighten, thereby placing the plurality of interlocking elements 20 into a fixed relationship with respect to each other. Accordingly, snake 18 is now in a selectively fixed position. Pivoting lever 16 interacts with an off-centered cam 28, and spring 30 to vary the tension in cable 26.

An endoscopic instrument holder or grasping mechanism 32 is disposed at distal end 14 of holder 10. Holder 32 includes a fixed, with respect to distal end 14, V-shaped notch 34 for receiving, for example, the handle of an endoscopic instrument (not shown). Holder 32 also includes an axially moveable clamp 36, which is fixedly connected to a rack 38.

In use, the handle of the endoscopic instrument is preferably placed between V-shaped notch 34 and clamp 36. The axial position of clamp 36 is moved toward notch 34 by the user turning or rotating thumb piece or handle 40. As thumb piece 40 is rotated, pawl 42 ratchets over gears 44 in rack 38. Pawl 42 is maintained in contact with gears 44 by a spring 46. The user will continue to rotate or actuate thumb piece 40 until the endoscopic handle is secured in place between notch 34 and clamp 36. To remove or adjust the position of the endoscopic handle, a quick release mechanism is provided. The user can simply depress a projecting end 48 of pawl 42 with sufficient force to overcome spring 46 to disengage the opposite end of pawl 42 from gears 44 of rack 38. Spring 50 biases rack 38 and clamp 36 away from V-shaped notch 34. Thus, once end 48 is depressed, clamp 36 quickly moves to an open position and releases the endoscopic handle.

The user can mount proximal end 12 of holder 10 to a ring or to a post as desired. If needed lever 16 can be moved to the open position to place snake 18 in any desired position so that the distal end of the endoscopic instrument is placed where the surgeon desires in or near the surgical site. Lever 16 is then moved to the closed position to fix the position of snake 18. As described above, the endoscopic instrument can now be placed between clamp 36 and V-shaped notch 34 and secured in position, or this step can be achieved previous to fixing the position of the snake, as desired by the user.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. An endoscopic instrument holder comprising:
an elongated bar having a proximal end and a distal end, a pivoting lever disposed at the proximal end, a grasping mechanism disposed at the distal end, a selectively flexible wire and ball joint assembly disposed between said proximal end and said distal end,
said lever being moveable to selectively place said bar in one of a fixed position and an adjustable position, in said adjustable position said bar is free to move into a position determined by a user, in said fixed position said bar is rigid, and
said grasping mechanism having a fixed clamp portion and a moveable clamp portion, a handle being disposed at the distal end of said bar, actuation of said handle causing movement of said moveable clamp portion with respect to said fixed clamp portion.

2. The endoscopic instrument holder according to claim 1, wherein said fixed clamp portion includes a V-shaped notch.

3. The endoscopic instrument holder according to claim 1, wherein said moveable clamp is axially moveable.

4. The endoscopic instrument holder according to claim 2, wherein said moveable clamp is axially moveable.

5. The endoscopic instrument holder according to claim 3, wherein said grasping mechanism includes a spring biased pawl and rack.

6. The endoscopic instrument holder according to claim 4, wherein said rack is fixedly connected to said moveable clamp.

7. The endoscopic instrument holder according to claim 5, wherein said pawl includes a projecting end to permit quick release of the grasping mechanism to an open position.
